# EUROPEAN PATENT APPLICATION

(11) **EP 0 582 231 A1**
(43) Date of publication of application: **09.02.1994**
(21) Application number: 93112242.8
(22) Date of filing: 30.07.1993
(51) Int. Cl.: G01N 33/558, G01N 33/76, G01N 33/569

(54) **Solid phase assay**

(30) Priority: 03.08.1992 US 924119
(71) Applicant: Becton Dickinson and Company, Franklin Lakes, New Jersey 07417-1880 (US)
(72) Inventor: Rosenstein, Robert W., Ellicott City, Maryland 21042 (US); Naqui, Ali, Bel Air, Maryland 21015 (US); Lovell, Stephen J., Towson, Maryland 21204 (US); Kearns, Kevin T., Cockeysville, Maryland 21030 (US)
(74) Representative: Bosotti, Luciano

(57) **Abstract**

A device and method for solid phase assay of an analyte employing capillary flow of reagents and/or sample in the porous solid phase. The device is designed for use in a horizontal position and comprises a portion (6,7,8) for combining analyte with tracer and means (3,12) for indicating when the assay is complete.

## Description

### REFERENCE TO RELATED APPLICATIONS

This is a continuation-in-part of copending application U.S. Serial No. 07/818,000, filed December 30, 1991, which is a continuation of U.S. Serial No. 07/031,023, filed March 27, 1987, now abandoned.

### FIELD OF THE INVENTION

The present invention relates to solid phase assays for analytes, in particular to specific binding assays employing capillary flow of reagents and/or sample in a porous solid support.

### BACKGROUND OF THE INVENTION

Specific binding assays such as immunoassays have been found to be of great value in a variety of clinical and research applications due to the specificity of the binding between ligand and receptor. Many different protocols and formats for such assays have therefore been developed, one of which involves conducting the binding assay on a piece of porous material which is usually in the form of a strip. This type of assay takes advantage of the capillary properties of the porous material to sequentially bring reaction components into contact with each other. This is often accomplished by positioning reaction components at predetermined locations along the strip so that liquid applied to one end moves by capillary migration along the strip, contacting the other reaction components in the desired sequence. Presence of an analyte is generally determined by detecting the signal from a detectable label included in the binding reaction between ligand and receptor.

Examples of immunoassays using these principles, often referred to as immunocapillary or immunochromatographic assays, can be found in the disclosures of WO 87/02774, EP 0 306 772, U.S. Patent No. 4,094,647, U. S. Patent No. 4,999,285, U.S. Patent No. 4,168,146, GB 2 204 398, U.S. Patent No. 4,943,522, U. S. Patent No. 5,037,736, U. S. Patent No. 5,075,078, U. S. Patent No. 5,096,837 and C. Glad and A. O. Grubb, Analytical Biochemistry, 85: 180-187 (1978).

### SUMMARY OF THE INVENTION

The present invention provides a porous solid support having a first portion which is contacted with a sample suspected of containing the analyte to be detected. Analyte which is present in the sample flows through the capillaries of the solid support and combines with a tracer which is reversibly bound to the solid support. The mixture of analyte and tracer is transported by capillary flow through the material of the solid support to a second portion where they contact and bind to an immobilized binder which binds specifically to 1) the analyte or 2) the analyte and the tracer. Unbound tracer moves through the second portion and into a third portion of the solid support by continued capillary flow. The third portion may serve only to receive materials not bound in the second portion (e.g., as in a sandwich assay) or it may contain additional reagents for detection of the unbound tracer (e.g., as in a competitive assay).

In the present preferred embodiment of the invention, the sample is applied to the first portion of the solid support and flows through the first portion into a separate portion containing tracer, thereby combining analyte which may be present in the sample with the tracer. Optionally, the combined analyte and tracer may then flow out of the tracer portion into an additional mixing portion of the solid support prior to being transported by capillary flow into the region of the solid support containing the second portion. This optional additional portion allows further mixing of the analyte and tracer and a period of incubation to allow binding. The third portion of the preferred embodiment includes an indicator zone containing a dye which is transported by the advancing fluid front to a position on the solid support where it can be viewed as an indication that the assay is complete.

### DESCRIPTION OF THE DRAWINGS

Fig. 1 is a perspective view of a representative device according to the invention.

Fig. 2 is a sectional view of a representative device taken along line 2 of Fig. 1.

Fig. 3 is a top plan view of the solid support showing placement of the openings in the upper section of the housing.

Fig. 4 is a top plan view of the solid support after successful completion of a positive assay.

Fig. 5 is a top plan view of a representative device showing an successfully completed positive assay.

### DETAILED DESCRIPTION OF THE INVENTION

The present invention is an embodiment of the invention disclosed in copending application U.S. Serial No. 07/818,000 (U.S. Patent No. ), the disclosure of which is hereby incorporated by reference. The present invention provides a preferred embodiment which represents a particular combination of certain of the specific structural and functional features of the solid support previously disclosed. In brief, the sample is applied to the first portion of the solid support with the solid support in an essentially horizontal position. The tracer portion is separate from the first portion and placed relative to the first portion such that fluid applied to the first portion flows vertically through the first portion and enters the tracer portion, after which the tracer and analyte are transported laterally by capillary flow to the second portion. The third portion of the preferred embodiment contains an additional feature for indicating that the capillary flow of the fluid (i.e., the fluid front) has advanced to a predetermined completion zone on the solid support, signalling that the assay is complete.

The region of the solid support containing the first portion comprises a macroporous material capable of absorbing a fluid sample applied to its surface. The sample passes through the capillary channels of the porous first portion into a separate tracer portion with which it is in fluid communication. Materials suitable for use in the first portion include porous polyethylene (e.g., POREX available from Porex Technologies, Fairburn, Georgia), glass fiber, rayon, nylon and cellulosic materials such as paper. Porous polyethylene is particularly preferred. Most preferably, the first portion comprises a layer having an upper and a lower surface. The sample is applied to the upper surface of the first portion and passes through it in a direction substantially perpendicular to the plane of the upper surface, exiting through the lower surface into the tracer portion. The first portion functions primarily as a filter for physical removal of particulate matter such as cells and debris which may be present in biological samples. When functioning only as a filter, the first portion may consist only of the macroporous material. However, reagents may also be added to the first portion as required for the assay. These reagents may be impregnated in the macroporous material of the first portion and include, for example, buffers and detergents.

The tracer portion is similarly comprised of a macroporous material such as porous polyethylene, glass fiber, rayon, nylon, or cellulosic materials such as paper. The tracer portion preferably comprises porous polyethylene. The tracer portion is in fluid communication with the first portion and absorbs sample fluid as it exits from the first portion. Preferably it is also a layer which has an upper surface contacting the lower surface of the first portion and flow of fluid through the capillary channels of the tracer portion is in a direction substantially perpendicular to the plane of its upper surface. The tracer portion further includes a tracer which comprises a detectable label conjugated to 1) a ligand for the analyte, 2) the analyte or 3) an analyte analog. A ligand for the analyte is a molecule which specifically recognizes and binds to the analyte, i.e., the ligand and the analyte are a specific binding pair. Many such ligands are known in the art, for example antigens and antibodies, enzymes and their substrates, carbohydrates and lectins, and avidin or streptavidin and biotin. For use in the present embodiment of the invention, antibody and antigen ligands are preferred.

The tracer is supported on or in the tracer portion of the solid support such that when fluid from the first portion contacts the tracer portion the tracer and analyte are transported together out of the tracer portion by capillary flow into the second portion. That is, the tracer is immobilized in the tracer portion but is reversibly bound and upon wetting of the tracer portion by sample fluid the tracer is released. The detectable label component of the tracer may be any detectable label known in the art for use in specific binding assays, particularly immunoassays. These include radioisotopes, fluorescent dyes, enzymes capable of reacting to produce colored products, visible dyes, etc. Methods for conjugating these labels to ligands and detecting the signals they produce are well known in the art.

Particulate detectable labels are preferred for use in the present embodiment of the invention. Suitable particles include particles of polymers (e.g., latex or polystyrene), sacs, liposomes, metallic sols (e.g., colloidal silver or colloidal gold) or polymeric dyes. To form the tracer, such particles are derivatized to include the detectable label, usually by formation of a chemical bond using methods known in the art for this purpose. In the case of sacs and liposomes, the label may also be entrapped in the vesicle. The particle and its associated label may be chemically conjugated to the ligand component of the tracer. Alternatively, polymer particles, polymeric dyes and metal particles may be coated with the ligand component of the tracer, e.g., as described in U.S. Patent No. 5,096,837. Most preferred for use as a detectable label in the present embodiment of the invention are polymeric particles impregnated with a visible absorbing dye, particularly 0.4-0.5 micron latex microparticles such as BLUE DYED LATEX available from Bangs Laboratories, Indianapolis, Indiana.

The present embodiment of the invention further includes an optional mixing portion in fluid communication with the tracer portion. The mixing portion also comprises a macroporous material as disclosed above for the first and tracer portions and is preferably porous polyethylene. Fluid passing through the first and tracer portions flows into the mixing portion prior to being transported out of the mixing portion into the segment of the solid support containing the second portion. The mixing portion facilitates mixing of the analyte with the tracer prior to capillary flow out of the mixing portion into the second portion. In addition, in sandwich assays the mixing portion provides a period of incubation for binding of analyte and tracer prior to contact of the analyte/tracer complex with the second portion. Preferably the mixing portion is also in the form of a layer with an upper surface in fluid communication with the lower surface of the tracer portion.

Fluid passing through the tracer portion enters the mixing portion through its upper surface and flows in a direction essentially perpendicular to the plane of the mixing portion layer, exiting through the lower surface. The mixing portion is in fluid communication with the segment of the solid support containing the second portion and fluid transport proceeds from the mixing portion into the second portion by capillarity. If the mixing portion is not included in the assay device, the tracer portion is in direct capillary fluid communication with the segment of the solid support containing the second portion.

The segment of the solid support containing the second portion comprises an absorbent microporous material. The microporous material serves to wick fluid from the preceding portions into the second portion by capillarity. That is, the fluid mixture of analyte and tracer is transported by capillary flow from the tracer portion or the mixing portion into the microporous material and continues to be transported within the microporous material into contact with the binder of the second portion. Suitable microporous materials for use in this segment of the preferred assay device include filter paper, chromatographic papers, glass fiber, crosslinked dextran, nylon and nitrocellulose. Nitrocellulose is most preferred because the binder of the second portion can be easily immobilized on this material without requiring covalent attachment.

While not required, most preferably the segment of the solid support comprising the absorbent microporous material is positioned such that the direction of capillary flow within it is substantially perpendicular to the direction of flow through the preceding portions, as shown in the accompanying Figures. This provides an assay which can be performed with the assay device in a horizontal rather than a vertical position, eliminating the need to hold the device during the performance and reading of the assay. In addition, the most preferred assay device includes all of the preceding portions, including the optional mixing portion.

The binder of the second portion is a ligand or receptor which specifically binds to a complementary binding pair member which is 1) the analyte or 2) the analyte and the tracer depending on whether the assay is performed in a sandwich or a competitive assay format. Many such binder/complementary binding pair member pairs are known in the art, and they include antibodies and antigens, lectins and carbohydrates, enzymes and their substrates, and specific binding proteins such as biotin and avidin or streptavidin. For use in the assay of the present invention, antigens or haptens and polyclonal or monoclonal antibodies are preferred binders for the second portion. The selection of antibody or antigen for the second portion binder depends on the nature of the analyte being detected, i.e., whether the analyte is itself an antigen or an antibody. When the analyte to be detected is an antibody the binder in the second portion may be the antigen specifically recognized and bound by that antibody. Alternatively, the antibody analyte may be bound in the second portion by a suitable anti-antibody antibody. Conversely, when the analyte to be detected is an antigen or hapten the binder in the second portion may be an antibody directed against that antigen.

The binder is immobilized on the microporous material in the second portion such that it is not removed from the second portion by the capillary flow of fluid through the second portion. That is, the binder is nondiffusively bound to the microporous material and remains immobilized in the second portion when the second portion is wetted by the fluid containing analyte and tracer. Such immobilization may be accomplished by covalent attachment of the binder to the microporous material using methods known in the art. Alternatively, satisfactory noncovalent attachment is possible when nitrocellulose is the microporous material. For this reason, nitrocellulose is the preferred material, as it allows for simpler methods of attachment of the binder. The binder may therefore be spotted or printed on the second portion when nitrocellulose is used, and is preferably applied in a defined pattern such as a "plus" sign, a checkmark or a geometric figure (e.g., a circle, square or triangle). As the fluid containing the analyte and tracer flows into the second portion the analyte and tracer become bound to the binder in the second zone and are immobilized there.

If a defined pattern of binder is immobilized in the second portion a second pattern of a second binder may also be applied at a location on the solid support which is adjacent to the second portion binder such that the two binders are contacted by the advancing fluid front under similar experimental conditions. This second pattern may be used as a positive control area to assure that the assay is functioning properly, containing either an antigen or antibody as appropriate. For example, if the assay is designed to detect an antigen analyte, the second pattern may comprise the antigen to be detected. If the assay is designed to detect an antibody analyte, the second pattern may comprise an anti-antibody. Alternatively, the binder of the second pattern may be unrelated to the analyte being detected, for example an anti-hapten antibody which binds a labeled hapten included in the tracer portion. Examples of suitable haptens include biotin, DNP and benzoic acid, with the corresponding antibodies being present in the second pattern of the second portion.

After contacting the second portion, the fluid continues to move by capillary migration through the microporous material, through and past the second portion and into the third portion. That is, the third portion is in capillary fluid communication with the second portion. The third portion comprises the area of the microporous support which receives fluid from the second portion. In a competitive assay format, the third portion may contain a substance for detecting tracer which was not bound in the second portion. In a sandwich assay format the third portion usually functions only to receive the fluid containing materials which were not bound in the second portion, i.e., it does not contain any additional reagents related to detection of analyte. Preferably, the third portion further includes an indicator zone of visible, water soluble dye such as Erythrosin B, Safranin O or Phenol Red applied to the support in a defined area such that the dye is reversibly bound to the support. When the fluid front has traversed the second portion and entered the third portion it flows through the indicator zone and the dye is carried by the fluid from its original position on the microporous material to a second position (the completion zone) where it can be visually detected as an indication that the fluid front has passed through the first, tracer and second portions and the assay is complete. If the third portion includes reagents for detection of unbound tracer, the indicator zone is located in an area contacted by the fluid front after contact with the reagents.

In an alternative embodiment, the tracer is reversibly immobilized on the microporous material in an area thereof defining the tracer portion. The tracer portion is placed between the first portion and the second portion containing the binder. The first portion is in direct fluid communication with the microporous material. A sample applied to the first portion is wicked into the microporous material where it contacts and mixes with the tracer. After flowing through the tracer portion the fluid continues to move by capillarity into the second portion for interaction with the binder. This embodiment has particular advantages in assays for antibody analytes which employ an antigen tracer, as less tracer can be used with resulting cost savings.

The most preferred assay protocol for the present preferred embodiment of the invention is a sandwich assay for detection of an antibody or antigen analyte. In a sandwich assay, because the tracer/analyte complex is immobilized and detected in the second portion, the binder is usually present in excess to ensure that substantially all of the complex is bound. Detection of the intensity of the label signal may then be used as a qualitative, quantitative or semiquantitative indication of the amount of analyte present.

By way of example, in a sandwich assay if an antibody is the analyte to be detected, the ligand portion of the tracer may be an antigen or hapten recognized and bound by the antibody analyte. Antigen or hapten may also be the binder immobilized in the second portion. One skilled in the art will recognize that in order to have binding in the second portion when the binder and tracer are hapten or analyte in a sandwich assay for detection of antibody, the tracer will be present in an amount which is below what would saturate the analyte binding capacity of the antibody. Such routine adjustments in the quantity of reagents are within the ordinary skill in the art. Alternatively, either the tracer or the binder may be an anti-analyte (Ab) antibody.

To perform the sandwich assay, a sample suspected of containing the antibody is applied to the first portion and allowed to move by capillary migration through the first portion into the tracer portion where it mixes with the labeled tracer. If present, the analyte antibody binds to the ligand component of the tracer. As the fluid moves through the tracer portion the tracer is transported out of the tracer portion with the fluid. Most preferably the mixture is transported out of the tracer portion into a mixing portion which allows additional time for binding of the antibody and tracer prior to contact with the microporous material and the second portion. In the second portion the binder binds to the antibody/tracer complex and immobilises it in the second portion. The label component of the tracer can then be detected in the second portion to indicate a positive assay result. If the assay is negative, i.e., there is no antibody present in the sample, no antibody/tracer complex will be bound in the second portion and no tracer will be detected. The fluid front containing any unbound antibody, tracer and antibody/tracer complex continues to move by capillarity through the second portion into the third portion where it contacts the dye localized in the indicator zone. The fluid front carries the dye from the indicator zone to the completion zone indicating that the fluid front has passed the second portion and the assay is complete. Representative examples of antibody analytes which may be detected using this method are antibody to human immunodeficiency virus (HIV), antibody to rubella, antibody to cytomegalovirus, antibody to Epstein Barr Virus (EBV) and antibody to the Lyme disease pathogen Borrelia burgdorferi.

Conversely, a sandwich assay in which an antigen analyte is to be detected may employ a tracer comprising an antibody which specifically recognizes and binds to the antigen being detected. The binder in the second zone may be a second anti-antigen antibody which recognizes and binds to an epitope on the antigen which is not the same as the epitope bound by the tracer antibody. Otherwise, the sandwich assay for antigen is performed essentially as described above for detecting an antibody analyte. Representative examples of antigen analytes which may be detected using this method are Group A Streptococcus antigens, human chorionic gonadotrophin (HCG), luteinizing hormone (LH) and Chlamydia antigens.

The assay may also be performed in a competitive assay format. In this embodiment the analyte (either antigen, hapten or antibody) is mixed with a tracer comprising a ligand which is capable of competing with the analyte for binding in the second portion. The binder is therefore usually present in an amount which will bind less than the total amount of analyte and tracer when analyte is present but is sufficient to bind essentially all of the tracer when analyte is absent. This allows unbound tracer to be detected in the third portion when analyte is present. In general, if the analyte is an antibody the ligand component of the tracer is the same antibody, but the tracer antibody may be any antibody which recognizes the same epitope as the analyte antibody or competes with it for binding to the binder. If the analyte is an antigen or hapten the ligand component of the tracer is usually the same antigen or hapten, but it may also be an analog of the analyte. An analog of the analyte antigen or hapten includes any derivatives of the antigen or hapten which are capable of competing with the analyte for binding to the binder in the second portion.

For a competitive assay, after mixing with the appropriate tracer in the tracer portion and, optionally, in the mixing portion the combined tracer and analyte are transported by capillary migration in the microporous support material into contact with the binder in the second portion. When the analyte is an antibody, the binder is usually the antigen or hapten recognized by the antibody or an analog thereof. It is usually an antibody which recognizes the antigen or hapten when antigen or hapten is the analyte. If an analog of the analyte is used as the tracer for an antigen or hapten analyte, the antibody binder also recognizes and binds to the analog.

The fluid front continues to be transported by the microporous material through and past the second portion, allowing competitive binding and immobilization of analyte and tracer in the second portion. Unbound tracer and unbound analyte then flow by capillarity into the third portion where the tracer contacts additional reagents, if required, and the label is detected. For example, if the label is an enzyme which produces a colored reaction product, the additional reagents would include the appropriate enzyme substrate. Labels such as fluorescent dyes, radioisotopes, and absorbing dyes are directly detectable without further reaction and usually no additional reagents in the third portion are required. The advancing fluid front also carries dye from the indicator zone into the completion zone of the solid support where it can be visualized, indicating that the fluid has traversed both the second portion and the reagent-containing area of the third portion, if present. This serves as an indication that the assay is complete.

Whether the assay format is a sandwich or competitive assay, when the tracer label is a dye it is preferred that the assay method include a step to stabilize color development in the portion where the tracer label is detected. This is because in immunocapillary assays continued capillary flow of fluid into the area where the label is detected allows the signal from the label to continue developing in intensity. This signal "drift" may cause a negative result to become a false positive result over time, or a weak positive signal to be come a strong positive. At present there is no method available which stops or stabilizes the signal in an immunoassay based on capillary migration. It has now been found, however, that application of a hygroscopic material to the solid support after addition of the sample inhibits signal drift and stabilizes signal development for later reading of the assay result. It is preferable to add the hygroscopic material to an area of the solid support upstream of the second portion and most preferable to add it at the site of addition of the sample.

Hygroscopic materials useful for stabilizing color development in the present assay include polysodium acrylate (PSA, available from United Desiccants, Pennsauken, New Jersey), SEPHADBX (a bead-formed gel prepared by cross-linking dextran with epichlorohydrin, available from Pharmacia Fine Chemicals, Piscataway, N.J.), and absorbent cellulose derivatives such as TRANSORB cellulosic filters (American Filtrona, Richmond, Virginia). Most preferred is SEPHADEX G-50. While not wishing to be bound by any specific theory of how this aspect of the invention operates, Applicants believe that the hygroscopic material stabilizes signal development by absorbing fluid from the porous support, thus interrupting capillary flow of fluid and tracer into the portion where label is to be detected. The selected hygroscopic material may be applied to the first portion of the solid support either after the fluid front has traversed the portion where the label is to be detected or it may be applied substantially immediately after addition of the sample. This color stabilizing method is particularly useful when the tracer label includes a visible dye, such as a dye conjugated to or incorporated in latex particles.

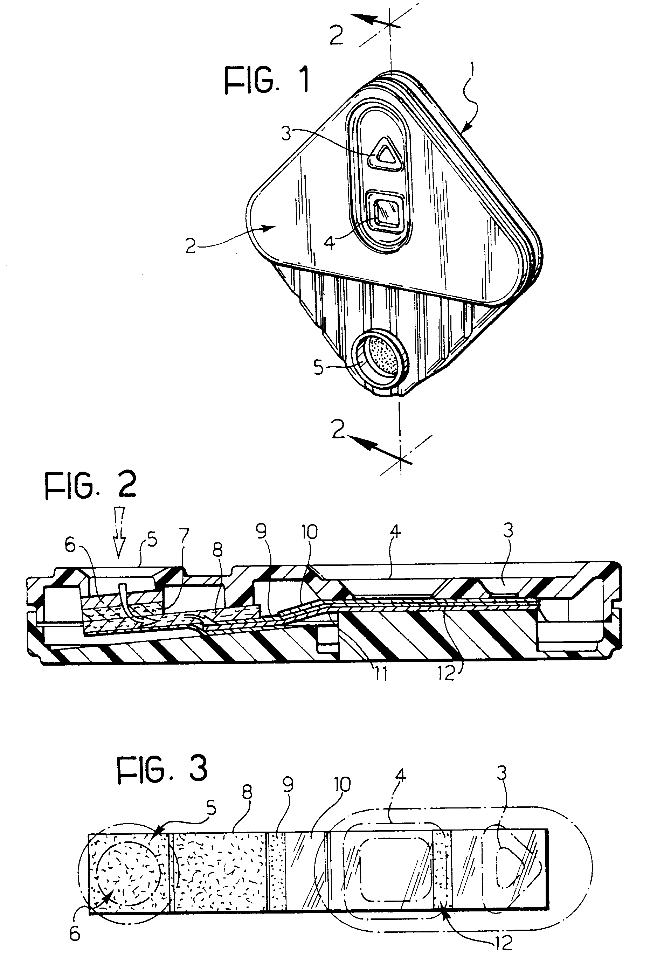

The materials and reagents described above are preferably packaged in the form of an assay device for ease of use. An example of such a device, to be used in a horizontal position, is illustrated in Fig. 1. The porous support with the first, tracer, mixing, second, third and indicator portions is placed within a housing of a suitable fluid-impermeable material. Moldable plastics such as polystyrene are preferred, but other impermeable materials such as glass or metal may also be used. The housing comprises a lower section (1) and an upper section (2). The porous support lays in the lower portion of the housing and is covered by the upper portion which has a plurality of openings therein. These openings are aligned with the porous support and provide visual or sample access to the various portions of the support (3-5). Referring now to Fig. 2, the sample port opening (5) provides access to the macroporous material comprising the first zone (6) and preferably has raised sides around the opening which form a well to facilitate application of a fluid sample to the first zone. The macroporous material comprising the first zone fits snugly against the interior edge of the sample port such that sample fluid applied thereto has access to the underlying tracer portion essentially only by flowing through the first portion. The macroporous materials comprising the tracer (7) and optional mixing (8) portions of the support are aligned with the sample port beneath the first zone such that fluid flow through the capillaries of these materials is substantially perpendicular to the plane of the device and the plane of the first portion.

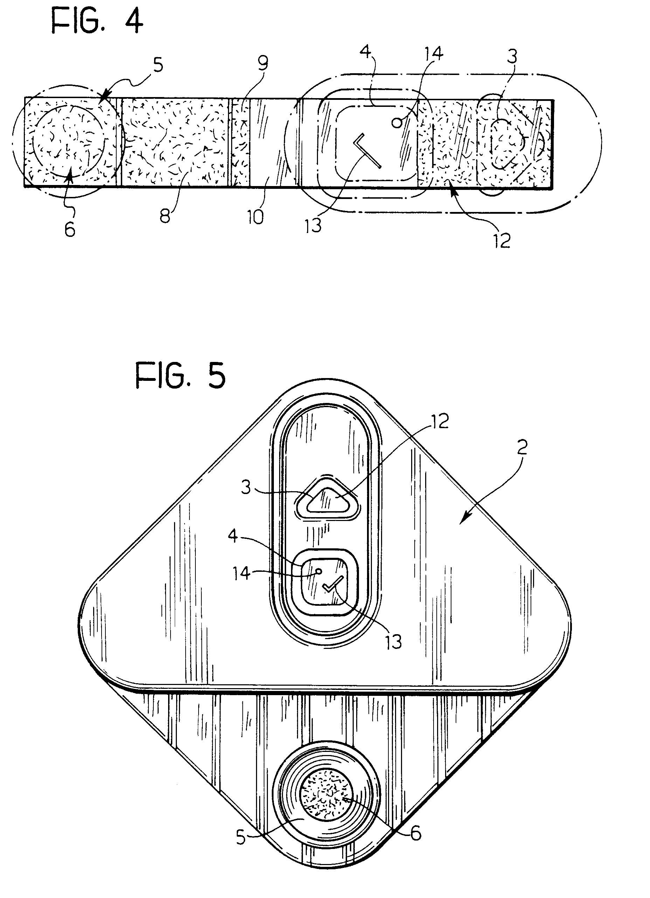

The microporous material carrying the second portion (9) contacts the macroporous material comprising the tracer portion or the mixing portion, if present, and extends within the housing in a direction substantially perpendicular to the direction of fluid flow through the first and tracer portions. Preferably, the microporous material is placed between an upper layer (10) and a lower layer (11) of a fluid impermeable material such as polyethylene terephthalate (e.g., MYLAR, available from Adhesives Research Incorporated, Glen Rock, Pennsylvania) or polyethylene. The microporous material is not covered with the fluid impermeable material where it contacts the tracer or mixing portions so that capillary flow of fluids in the device is not disrupted. In the sandwich assay device illustrated, a detection window (4) is provided in the upper section of the housing aligned with the second portion of the microporous material and providing visual access thereto. The detection window allows visualization or detection of tracer bound to the second zone as shown in Fig. 4 (13), indicating a positive result in a sandwich assay. In a device for use in competitive assays, the detection window may be placed in alignment with the area of the third portion where unbound tracer can be detected.

The indicator dye (12) is located in the indicator zone on the microporous support at a position which is contacted by the fluid after it passes through the second portion. Alternatively, the indicator dye may be in an area of the microporous support which is contacted by the fluid after passing through the second portion and the area of the third portion where unbound tracer is detected in a competitive assay. The indicator zone is preferably concealed under the upper section of the housing and not visible prior to running an assay using the device. When an assay is run and the fluid front passes through the indicator zone, the dye is moved by capillary flow along the support into alignment with a completion window (3) in the upper section of the housing, at which time the dye becomes visible through the completion window. This indicates that the fluid front has passed through all portions of the device required for successful completion of the assay. Fig. 4 illustrates a completed positive assay in which the indicator dye (12) has moved into view in the completion window (3), with tracer immobilized in the second portion (13) and in the positive control area (14). Fig. 5 illustrates the positive assay of Fig. 4 as it would be viewed by the Practitioner using the intact device.

The following experimental Examples are provided to illustrate certain features of the invention but are not to be considered as limiting the scope of the invention as defined by the appended claims.

### EXAMPLE 1

### CONSTRUCTION AND USE OF AN ILLUSTRATIVE ASSAY DEVICE

An approximately 9 X 10 mm piece of porous polyethylene (15-45 µm POREX) approximately 1.5 mm thick was impregnated with a tracer comprising anti-HCG antibody labeled with dye impregnated 0.4-0.5 micron latex microparticles (BLUE DYED LATEX, Bangs Laboratories, Indianapolis, Indiana) to form a tracer portion. The tracer portion was sandwiched between a second 9 X 10 mm piece of porous polyethylene and a 9 X 19mm piece of porous polyethylene and the polyethylene stack was placed on top of a strip of nitrocellulse approximately 9 X 46 mm in size with the bottom edges aligned. The polyethylene stack and the nitrocellulose were held together with adhesive backed MYLAR (AS-110 ACRYLIC SYSTEM from Adhesives Research Inc., Glen Rock, Pennsylvania) placed along the aligned bottom edge. Approximately 10-15 mm from the free edge of the larger piece of polyethylene 1 microgram of anti-HCG antibody in 1 µl of PBS/5% glucose was spotted on the nitrocellullose in the shape of a checkmark and allowed to dry. 5 µg of HCG in 1 µl of PBS were spotted in a small dot adjacent to the anti-HCG antibody checkmark as a positive control area and also allowed to dry. 1 µl of Erythryosin B (Aldrich Chemical Co., Inc., Milwaukee, Wisconsin) was placed in a band across the nitrocellulose about 5 mm above the anti-HCG binder (i.e., about 15-20 mm from top edge of the larger piece of polyethylene). The prepared solid support was then placed in the lower section of a molded polystyrene housing as shown in the Figures. The upper section of the housing with openings aligned with the smaller piece of polyethylene, the anti-HCG checkmark/HCG dot, and an area of the nitrocellulose downstream from the band of Erythrosin B was placed over the support and snapped into place on the lower section.

To perform an assay for HCG using the device, five drops (about 320 µl) of urine were added to the sample well and absorbed. After about 1 min. the Erythrosin B indicator dye appeared in the completion window, indicating completion of the assay. At that time a blue checkmark appeared in the detection window, indicating a positive result for HCG. The blue dot adjacent to the checkmark was also visible as a positive control. Negative control samples consisted of pooled negative urine or PBS, and when these were run in the assay system only a blue positive control dot appeared in the detection window.

### EXAMPLE 2

### STABILIZATION OF COLOR DEVELOPMENT

A device similar to the device shown in the Figures for assaying HCG was used to test methods for stabilization of color development. The assay was a sandwich assay in which the tracer and binder comprised anti-HCG antibodies and the tracer label was BLUE DYED LATEX.

Five drops of urine samples containing 250 mIU/ml of HCG were added to the sample port of the assay devices and the assay was allowed to run until the dye became visible in the indicator window, indicating the end of the assay. At that time SEPHADEX G-50 was added to the sample port to fill it. Control assays were performed in which no SEPHADEX was added to the sample port. Negative control assays were run with 5 drops of urine which did not contain HCG.

Assay results were scored visually on a scale of 1+ to 4+. For positive samples, the color reaction was initially evaluated as 1-2+. This intensity was maintained for four days in the devices which were treated with SEPHADEX. In contrast, when no SEPHADEX was added to the positive assay devices, the signal became progressively stronger over four days.

For the negative controls, the initial test result was negative. However, when no SEPHADEX was added to the assay device a false positive result developed between 1 and 4 days after completion of the assay. Devices treated with SEPHADEX remained negative for four days.

Similar experiments assaying for Group A Streptococcus antigens were performed to test the stabilizing capabilities of PSA and FILTRONA cellulose filters. In this instance, the hygroscopic material was added to the sample port substantially immediately after addition of the sample to the assay device. The samples were nitrous acid extracts of throat swabs which tested negative for Group A Strep by the latex test (CULTURETTE 10 MINUTE, Becton Dickinson Advanced Diagnostics, Baltimore, Maryland).

Throat swabs negative for Group A Streptococcus organisms were extracted with nitrous acid. Five drops (about 320 µl) of this extract were added to the sample wells of the assay devices. As soon as the liquid was absorbed, the absorbent material (PSA or TRANSORB) was added to the well. In control assays, no absorbent was added. Assay results were monitored visually as 1+ to 4+ in the detection window at the time the indicator dye appeared in the completion window (End of Assay, "EOA") and 3, 5, and 10 min. later. Samples without added absorbent showed a significant proportion which drifted from negative to positive within the experimental time period. In contrast, samples with added PSA or TRANSORB showed no increase in false positives.

To determine the effect of color stabilization on sensitivity of the assay, 5 drops of extracted Group A Streptococcus antigen were added to assay devices and the results compared at EOA with and without PSA or TRANSORB. No detectable difference in sensitivity due to the absorbent was found.

## Claims

1. A device comprising a solid support for detecting an analyte in a fluid, the support comprising:
a) a first portion comprising a layer of macroporous material;
b) a tracer portion in fluid communication with the first portion such that fluid flow between the first portion and the tracer portion is in a direction substantially perpendicular to the plane of the first portion layer, the tracer portion comprising a layer of macroporous material having a tracer reversibly immobilized therein;
c) a microporous material in fluid communication with the tracer portion such that the microporous material wicks fluid from the tracer portion in a direction substantially perpendicular to the direction of fluid flow in the first and tracer portions;
d) a binder for the analyte nondiffusively immobilized on the microporous material in an area defining a second portion; and
e) a visible dye reversibly bound to the microporous material in an indicator zone contacted by the fluid after contact of the fluid with the second portion.

2. The device according to Claim 1 wherein the macroporous material of the first and tracer portions is porous polyethylene and the microporous material in nitrocellulose.

3. The device according to Claim 1 wherein the support further comprises a mixing portion in fluid communication with the tracer portion such that fluid flow between the tracer portion and the mixing portion is in a direction substantially perpendicular to the plane of the tracer portion layer, the mixing portion comprising a layer of macroporous material.

4. The device according to Claim 3 wherein the macroporous material of the first portion, the tracer portion and the mixing portion is porous polyethylene.

5. The device according to Claim 4 wherein the microporous material is nitrocellulose.

6. The device according to Claims 1 or 3 wherein the solid support is enclosed in a fluid-impermeable housing having an upper section including a first opening aligned with the first portion, a second opening aligned with the second portion, and a third opening aligned with an area of the microporous material contacted by the fluid after it flows through the indicator zone.

7. The device according to Claim 1 wherein the tracer comprises a label including a dye-coated particle.

8. The device according to Claims 1 or 3 wherein the tracer and binder comprise antibodies which bind to the analyte.

9. The device according to Claims 1 or 3 wherein the solid support is enclosed in a fluid-impermeable housing having an upper section including a first opening aligned with the first portion, a second opening aligned with a third portion of the microporous support which is in fluid communication with the second portion and is contacted by the fluid after it flows through the second portion, and a third opening aligned with an area of the microporous material contacted by the fluid after it flows through the indicator zone.

10. The device according to Claims 1 or 3 wherein the tracer is the analyte or an analog thereof and the binder is an antibody which binds to the analyte.
